# FASCICULE DE BREVET EUROPEEN

(11) **EP 4 505 289 B1**
(45) Date de publication et mention de la délivrance du brevet: **04.02.2026**
(21) Numéro de dépôt: 23716544.4
(22) Date de dépôt: 03.04.2023
(51) Int. Cl.: G06F 8/60, G06F 9/445, G16H 30/20

(54) **PROCÉDÉ D'INTÉGRATION ET D'EXÉCUTION D'UNE APPLICATION MIS EN OEUVRE PAR UNE PLATEFORME D'IMAGERIE MÉDICALE**
VERFAHREN ZUR INTEGRATION UND AUSFÜHRUNG EINER ANWENDUNG, DIE VON EINER MEDIZINISCHEN BILDGEBUNGSPLATTFORM IMPLEMENTIERT WIRD
METHOD FOR INTEGRATING AND EXECUTING AN APPLICATION IMPLEMENTED BY A MEDICAL IMAGING PLATFORM

(30) Priorité: 04.04.2022 FR 2203056
(43) Date de publication de la demande: 12.02.2025
(73) Titulaire: Olea Medical, 13600 La Ciotat (FR)
(72) Inventeur: LOPEZ, Bruno, 13600 LA CIOTAT (FR); PETER, Philippe, 83330 LE BEAUSSET (FR)
(74) Mandataire: Brun, Philippe Alexandre Georges
(86) Numéro de dépôt international: PCT/EP2023/058715
(87) Numéro de publication internationale: WO 2023/194323

(56) Documents cités:
- US-A1- 2009 328 028
- US-A1- 2019 108 905
- US-A1- 2021 042 141
- MCGEE W C: "Data description for data independence", SIGMOD RECORD, ACM, NEW YORK, NY, US, vol. 1, no. 2, December 1969 (1969-12-01), pages 3 - 10, XP058332227, ISSN: 0163-5808, DOI: 10.1145/983038.983039

## Description

L'invention concerne un procédé d'intégration et d'exécution d'une application d'analyse d'images médicales mis en œuvre par une plateforme d'imagerie médicale.

Il est connu d'utiliser une plateforme d'imagerie médicale pour aider un praticien à identifier des pathologies en exploitant des images médicales acquises éventuellement selon différentes modalités par exemple via un appareil d'imagerie par résonance magnétique, également connue sous l'acronyme français IRM ou anglo-saxon MRI (pour « *Magnetic Resonance Imaging* »). Une telle plateforme d'imagerie médicale est conçue pour héberger une ou plusieurs applications logicielles généralement spécialisées pour identifier une pathologie donnée (accident vasculaire cérébral, pneumopathie, tumeur, etc.), analyser un organe déterminé (cerveau, poumon, sein, etc.) ou pour estimer des paramètres physiologiques ou hémodynamiques spécifiques (densité tissulaire, flux ou volumes sanguins, etc.). En milieu hospitalier, les images médicales sont générées depuis un ou plusieurs appareils d'imagerie médicale (IRM, scanners, etc.) et sont transmises auxdites plateformes ou archivées au sein de serveurs d'archivage et de transmission d'images également connus sous l'abréviation « PACS » de la dénomination anglo-saxonne « *Picture Archiving and Communication System* »*.* Les images médicales sont en outre encodées et transmises selon un protocole spécifique, connu sous le terme « DICOM » acronyme anglo-saxon de « *Digital Imaging and Communications in Medicine* », pour faciliter les transferts d'images médicales entre les appareils et systèmes d'imagerie de différents constructeurs.

Une plateforme d'imagerie médicale héberge ainsi une pluralité d'applications logicielles pour traiter une pluralité de cas (en termes de pathologies, organes, modalités d'acquisition). Le format DICOM permet d'associer des méta-informations complémentaires aux images en tant que telles à partir desquelles une plateforme d'imagerie médicale « décide » de lancer l'exécution de telle ou telle application logicielle. Ainsi, le document US 2019/0108905A1, décrit un mode de réalisation selon lequel, en fonction desdites méta-informations DICOM, un fichier de configuration d'enchaînement de la mise en œuvre de plusieurs applications (dénommées filtres) installées sur une plateforme, peut être sélectionné parmi une pluralité de fichiers de configuration, voire être créé, pour définir le processus de traitement de telles images médicales.

Les plateformes actuellement disponibles ne peuvent toutefois comporter à tout instant l'ensemble des applications logicielles pour répondre à une diversité de cas en croissance constante, ni même les toutes dernières versions desdites applications logicielles pour bénéficier continuellement des nombreuses innovations dans ce domaine. Il est donc nécessaire d'enrichir ou de mettre à jour la pluralité d'applications logicielles au sein des plateformes d'imagerie médicale déployées sur les sites.

D'un point de vue technique, une plateforme d'imagerie médicale s'apparente à un ordinateur comportant une unité de traitement consistant en un ou plusieurs microprocesseurs ou microcontrôleurs mettant en œuvre des instructions de programmes d'un système d'exploitation de ressources matérielles (mémoires de données ou de programmes, périphériques, etc. ) et des applications logicielles hébergées. Lesdites instructions de programme sont chargées dans une ou plusieurs mémoires de ladite plateforme en communication avec ladite unité de traitement.

On entend par « mémoire ou moyen de mémorisation » toute mémoire informatique que celle-ci soit volatile ou non. Une mémoire non volatile est une mémoire informatique dont la technologie permet de conserver ses données en l'absence d'une alimentation en énergie électrique. Elle peut contenir des données résultant de saisies, de calculs, de mesures et/ou des instructions de programmes. Les principales mémoires non volatiles actuellement disponibles sont de type inscriptible électriquement, telles que l'EPROM (« *Erasable Programmable Read-Only Memory* », selon une terminologie anglo-saxonne) ou encore inscriptibles et effaçables électriquement, telle que l'EEPROM (« *Electrically-Erasable Programmable Read-Only Memory* »), flash, SSD (« *Solid-State Drive* », selon une terminologie anglo-saxonne), etc. Les mémoires non volatiles se distinguent des mémoires dites « volatiles » dont les données sont perdues en l'absence d'une alimentation électrique. Les principales mémoires volatiles actuellement disponibles sont de type RAM (« *Random Access Memory* » selon une terminologie anglo-saxonne ou encore nommée « mémoire vive »), DRAM (mémoire vive dynamique, nécessitant une réactualisation régulière), SRAM (mémoire vive statique nécessitant une telle réactualisation lors d'une sous-alimentation électrique), DPRAM ou VRAM (particulièrement adaptées à la vidéo), etc.

Les plateformes d'imageries médicales connues présentent ou imposent des modes d'intégration des applications logicielles. Le mode d'intégration le plus répandu exploite le standard DICOM. Selon ce mode, toute application logicielle est conçue pour ne traiter que des données d'entrée au format DICOM et pour ne délivrer que des résultats selon ce même format, lesdites données devant en outre respecter un protocole de transmission spécifique. Ce premier mode d'intégration présente toutefois des inconvénients majeurs. Tout d'abord, le format et le protocole DICOM ne sont pas optimaux pour produire et partager un rapport à partir de résultats intermédiaires produits par d'autres applications logicielles. En outre, le standard DICOM ne permet pas de pouvoir configurer aisément des applications logicielles selon certains contextes de réception et/ou d'exploitation des données d'imagerie à analyser.

D'autres plateformes connues ont opté pour un deuxième mode d'intégration basé sur l'exploitation d'interfaces de programmation d'applications également connues sous l'abréviation API de l'expression anglo-saxonne « *Application Programming Interface* ». Cette solution est avantageuse car elle facilite l'accès aux services de configuration pour lesdites applications logicielles et ouvre de nombreuses possibilités d'exportation de résultats. En revanche, ce deuxième mode présente généralement un inconvénient majeur induit par le fait qu'une API est généralement spécifique ou dédiée à une plateforme d'imagerie médicale donnée ou à un constructeur donné de telles plateformes. Pour pouvoir développer une application intégrable sur une première plateforme d'imagerie médicale, le concepteur de l'application doit connaître l'API requise par ladite première plateforme et construire son application autour de cette API. Si ladite application doit être intégrée sur une deuxième plateforme requérant une deuxième API distincte de la première, ledit concepteur doit modifier la conception de son application et produire une deuxième application logicielle. En outre, il est délicat voire impossible pour ledit concepteur de tester le bon fonctionnement d'une application avant son émission en l'absence d'une plateforme de test tenue à sa disposition. Il doit pour cela disposer d'un émulateur de ladite plateforme ou d'un « bouchon » pour simuler le comportement de celle-ci, ce qui n'est pas toujours le cas ou peut s'avérer complexe ou onéreux.

Tout au plus, un développeur d'applications peut apporter une certaine indépendance au code « métier » d'une application au regard du format des données manipulées grâce à une génération d'une description desdites données, transmise concomitamment avec lesdites données manipulées, comme l'indique le document « Data description for data independence » de W.C. McGee, SIGMOD RECORD, ACM, NEW YORK, NY, US, vol. 1, no. 2, décembre 1969 (1969-12), pages 3-10, ISSN: 0163-5808, DOI: 10.1145/983038.983039, technique aujourd'hui mieux connue sous l'appellation « architecture hexagonale ». Toutefois, un tel enseignement n'apporte aucune solution pour conférer une indépendance de ladite application au regard des plateformes hôtes.

L'invention répond aux inconvénients soulevés par l'état de la technique.

Parmi les nombreux avantages procurés par l'invention, nous pouvons mentionner plus particulièrement que l'invention permet de concevoir des applications logicielles indépendamment d'une plateforme d'imagerie médicale particulière. Grâce à l'invention, lesdites applications logicielles peuvent exploiter et/ou produire des données conformes au standard DICOM ou selon tout autre format. Ainsi, les applications logicielles sont intégrées dans une plateforme d'imagerie médicale sous la forme de modules applicatifs autonomes spécifiant leurs dépendances dont les entrées et sorties sont lues ou écrites sur le système de fichiers qu'opère toute plateforme d'imagerie médicale. L'invention procure ainsi une très grande interopérabilité ne liant plus, comme c'est encore le cas aujourd'hui, une application à un type de plateforme donné. En outre, un concepteur d'une application a la capacité de tester très facilement le fonctionnement de son application avant l'émission de celle-ci sans nécessiter l'acquisition ou l'immobilisation d'une plateforme d'imagerie médicale à des fins de déverminage, d'un simulateur/émulateur de celle-ci ou encore d'un bouchon sophistiqué ou en variante, trop limité pour simuler l'intégralité des fonctionnalités d'une telle plateforme.

Par la suite du document, on entend par « système de fichiers » également connu sous la terminologie anglo-saxonne « *File System* », les services opérés par une plateforme d'imagerie médicale ou par tout ordinateur, qui déterminent l'organisation des données accessibles en lecture ou en écriture au sein d'une mémoire, ou plus généralement au sein d'un volume physique ou logique d'une telle mémoire. Classiquement, un tel système de fichiers détermine la manière de stocker lesdites données et de les organiser dans des structures logiques, que l'on nomme « fichiers ». Un tel système de fichiers permet de traiter, conserver et partager des données entre plusieurs programmes informatiques tels qu'un système exploitation des ressources de l'ordinateur (ou en l'espèce d'une plateforme d'imagerie médicale) et les applications logicielles qui font appel auxdites ressources via ledit système d'exploitation. Un système de fichiers offre ainsi une vue abstraite des données stockées dans une mémoire et permet d'y accéder en lecture et/ou en écriture à partir d'un chemin d'accès également nommé « *path* » selon une terminologie anglo-saxonne.

L'invention est spécifiée par les revendications indépendantes annexées. En outre, des modes de réalisation préférés sont définis par les revendications dépendantes.

A cette fin, l'invention prévoit un procédé d'intégration et d'exécution d'une application logicielle, ledit procédé étant conçu pour être mis en œuvre par une plateforme d'imagerie médicale opérant un système de fichiers. Un tel procédé comporte :
- une étape de chargement d'une application logicielle dans une mémoire de la plateforme d'imagerie médicale;
- une étape d'écriture de la valeur de la donnée d'entrée de l'application logicielle dans un répertoire d'entrée du système de fichiers;
- une étape de lancement de l'exécution de l'application logicielle;
- une étape de lecture de la valeur de la donnée de sortie de l'application logicielle dans le répertoire de sortie du système de fichiers;
   ledit procédé étant caractérisé en ce que ledit procédé comporte en outre:
   a. une étape de chargement d'un contrat dédié à ladite application logicielle dans ladite mémoire de la plateforme d'imagerie médicale, ledit contrat consistant en un contenu numérique non modifié ou configuré par ladite plateforme d'imagerie médicale, qui comporte des informations exploitables par cette dernière et imposées par l'application logicielle, lesdites informations décrivant :
      i. un identifiant unique désignant ladite application logicielle qui est ainsi associée au contrat qui lui est dédié;
      ii. un répertoire virtuel d'entrée destiné à contenir une valeur d'une donnée d'entrée nécessaire à l'exécution de l'application logicielle;
      iii. un répertoire virtuel de sortie destiné à contenir la valeur de la donnée de sortie produite par cette dernière, ladite étape de chargement dudit contrat étant réalisée conjointement avec le chargement de l'application logicielle qui lui est associée dans la mémoire de la plateforme d'imagerie médicale;
   b. une étape de lecture du contrat dédié à l'application logicielle qui lui est associée par la présence de l'identifiant unique de cette dernière inscrit dans ledit contrat, pour connaître les exigences de ladite application logicielle pour être exécutée, ladite étape de lecture du contrat précédant l'étape de lancement de l'exécution de ladite application logicielle;
- l'application logicielle est un code exécutable conçu et produit indépendamment de la mise en œuvre du présent procédé d'intégration et d'exécution ou de tout autre procédé mis en œuvre par l'unité de traitement de ladite plateforme d'imagerie médicale;
- l'étape de lancement de l'exécution de l'application logicielle désignée par ledit identifiant unique tiré du contrat lu préalablement, comprend une transmission d'arguments d'exécution de l'application logicielle consistant en des chemins d'accès au répertoire d'entrée du système de fichiers et au répertoire de sortie dudit système de fichiers, lesdits chemins d'accès étant ainsi associés respectivement aux répertoires virtuels d'entrée et de sortie de l'application logicielle grâce auxdits arguments d'exécution transmis à l'application logicielle à l'étape de lancement de l'exécution de cette dernière conformément au contenu du contrat préalablement lu à l'étape de lecture dudit contrat dédié à ladite application logicielle.

Pour permettre de paramétrer aisément l'exécution d'une application logicielle, le contrat peut comporter une valeur d'un paramètre de fonctionnement de ladite application logicielle. Dans ce cas, l'étape de lancement de l'exécution de ladite application logicielle d'un procédé conforme à l'invention peut être adaptée pour comporter comme argument la valeur dudit paramètre de fonctionnement.

Pour réaliser l'intégration d'une application logicielle, un procédé selon l'invention comprends une étape de chargement de l'application logicielle et du contrat dans une mémoire de la plateforme d'imagerie médicale.

De manière avantageuse et faciliter notamment le test préalable d'une application logicielle, cette dernière peut se présenter sous la forme d'un conteneur informatique, l'identifiant unique désignant ladite application logicielle compris dans ledit contrat exprimant l'image dudit conteneur informatique.

A titre avantageux, le répertoire virtuel d'entrée destiné à contenir la donnée d'entrée de l'application logicielle peut être associé dans le contrat à un attribut précisant le caractère optionnel ou obligatoire de ladite donnée d'entrée. Dans ce cas, lorsqu'un tel attribut atteste un caractère optionnel de ladite donnée d'entrée, l'étape d'écriture de cette dernière peut n'être mise en œuvre que si une telle donnée d'entrée est disponible pour la plateforme d'imagerie médicale.

L'invention prévoit également d'enrichir en méta-informations une donnée de de sortie produite par une application logicielle intégrée et exécutée selon l'invention. Pour cela, le répertoire virtuel de sortie destiné à contenir la valeur de la donnée de sortie de l'application logicielle peut être associé dans le contrat à un attribut de typage de ladite sortie. L'étape de lecture de la valeur de la donnée de sortie de l'application logicielle peut alors consister à associer ladite valeur dudit attribut de typage à la valeur de ladite donnée de sortie.

Pour préserver toute modification malveillante d'un contrat associé à une application logicielle, ledit contrat peut être encodé préalablement à son intégration dans la plateforme, voire après toute mise à jour de ce dernier par ladite plateforme. Dans ce cas, l'étape de lecture d'un contrat peut comporter une sous-étape préalable de décodage dudit contrat.

Selon un deuxième objet, l'invention concerne un produit programme d'ordinateur comportant des instructions de programme exécutables par l'unité de traitement d'un ordinateur, lesdites instructions de programme étant chargeables dans une mémoire non volatile dudit ordinateur et dont l'exécution par ladite unité de traitement provoque la mise en œuvre d'un procédé d'intégration et d'exécution d'une application logicielle selon l'invention.

Selon un troisième objet, l'invention concerne en outre un support de mémorisation lisible par un ordinateur comportant les instructions d'un tel produit programme d'ordinateur.

Enfin, l'invention concerne en outre une plateforme d'imagerie médicale comprenant une unité de traitement, une mémoire exploitée par un système de fichiers, ladite mémoire comprenant les instructions de programme d'un produit programme d'ordinateur selon l'invention.

D'autres caractéristiques et avantages apparaîtront plus clairement à la lecture de la description qui suit et à l'examen des figures qui l'accompagnent parmi lesquelles :
- la figure 1 illustre un système d'imagerie médicale comprenant une plateforme d'imagerie médicale ;
- la figure 2 illustre l'architecture simplifiée d'une plateforme d'imagerie médicale conforme à l'invention ;
- la figure 3 illustre un exemple de conception d'une application logicielle intégrable et exécutable par une plateforme d'imagerie médicale conforme à l'invention ;
- la figure 4 illustre un procédé d'intégration et d'exécution d'une application logicielle par une plateforme d'imagerie médicale conforme à l'invention.

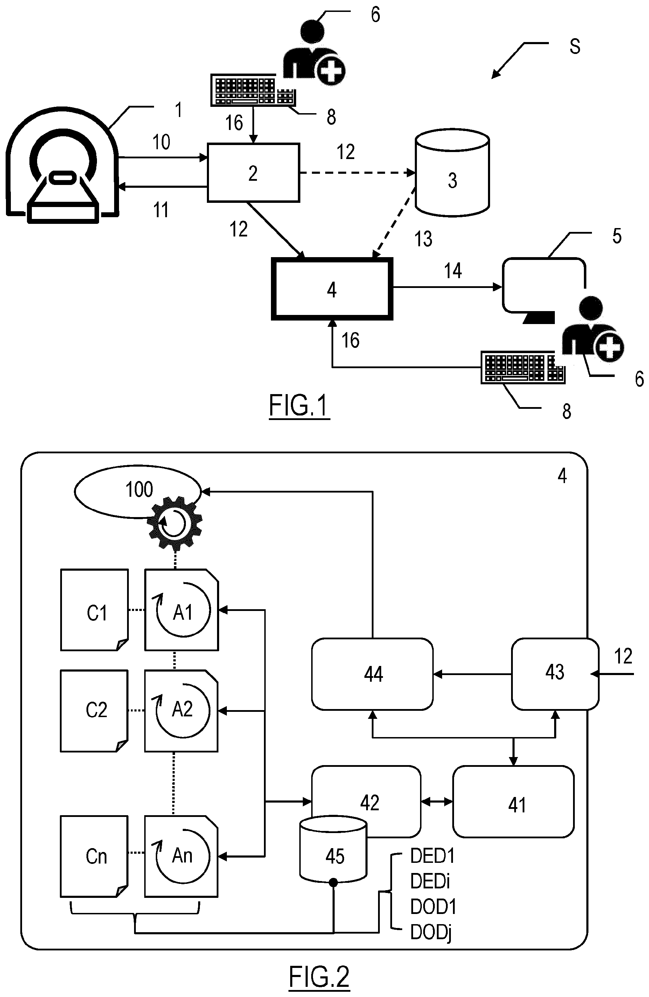

La figure 1 illustre un exemple d'un système d'imagerie médicale S comportant un appareil d'imagerie 1. Celui-ci délivre une pluralité de séquences d'images numériques 12 d'une ou plusieurs parties du corps d'un patient, à titre d'exemples non limitatifs, le cerveau, le cœur, les poumons. L'appareil d'imagerie par résonance magnétique 1 est généralement commandé à l'aide d'une console 2. Un utilisateur 6, par exemple un opérateur, praticien ou chercheur, peut ainsi choisir des commandes 11 pour piloter l'appareil d'imagerie 1 à partir de paramètres ou de consignes 16 saisis via une interface homme-machine d'entrée 8 du système d'analyse S. Une telle interface homme-machine 8 peut consister par exemple en un clavier informatique, un dispositif de pointage, un écran tactile, un microphone ou, plus généralement, toute interface agencée pour traduire une gestuelle ou une consigne émise par un humain 6 en données de commande ou de paramétrage d'un appareil d'imagerie 1. A partir d'informations 10 produites par ledit appareil 1, on obtient une pluralité de séquences d'images numériques 12 d'une partie d'un corps d'un humain ou d'un animal.

Les séquences d'images 12 peuvent optionnellement être stockées au sein d'un serveur 3 ou PACS, c'est-à-dire un ordinateur doté de moyens de mémorisation propres et constituer un dossier médical 13 d'un patient. Un tel dossier 13 peut comprendre des images de différents types, telles que des images fonctionnelles mettant en évidence l'activité des tissus ou des images anatomiques reflétant les propriétés des tissus. Les séquences d'images 12 ou, plus généralement les données expérimentales, sont analysées par une plateforme d'imagerie médicale 4 agencée à cette fin pour produire des indicateurs textuels ou graphiques 14 visualisables via une interface homme-machine de sortie 5 par un personnel de santé 6 selon des consignes 16 adressées à ladite plateforme d'imagerie médicale 4.

La figure 2 décrit un exemple d'architecture d'une plateforme d'imagerie médicale 4 conforme à l'invention et destinée à être intégrée dans un système d'analyse d'imagerie tel que le système S illustré par la figure 1. Une telle plateforme d'imagerie médicale 4 peut comporter une unité de traitement sous la forme d'un ou plusieurs microprocesseurs ou microcontrôleurs 41 mettant en œuvre des instructions de programmes d'applications idoines chargées dans des moyens de mémorisation ou mémoire 45 dudit système d'analyse d'imagerie S. L'accès en lecture et/ou écriture à ladite mémoire 45 par l'unité de traitement 41 peut être régi par un système de fichiers 42. Ladite unité de traitement 41 est avantageusement en communication avec un module d'entrée/sortie 43 chargé notamment de réceptionner les séquences d'images médicale 12, voire avec un module 44 chargé de déterminer, à partir desdites séquences d'images 12 réceptionnées, quelle est ou quelles sont les applications logicielles parmi des applications logicielles disponibles A1, A2, An qui méritent d'être mises en œuvre pour analyser lesdites séquences d'images 12 dans le but de produire des quantités d'intérêt pertinentes 14 à destination des personnels de santé 6. Lesdites applications logicielles A1 à An sont préalablement, ou plus précisément les instructions de programmes qui les caractérisent, chargées en mémoire 45 de la plateforme 4. Comme nous l'étudierons ultérieurement chaque application logicielle A1, A2, An est associée à un contenu numérique distinct de ladite application logicielle et qui est dédié à cette dernière, contenu numérique que nous nommons dans la suite « contrat » C1, C2, Cn. Ainsi, conjointement au chargement des instructions de programme de l'application logicielle A1, un contrat C1 sous la forme d'un contenu numérique comportant des informations exploitables ou interprétables par l'unité de traitement 41, plus précisément selon un procédé 100 d'intégration et d'exécution d'applications logicielles conforme à l'invention. Pour adapter le fonctionnement de la plateforme d'imagerie médicale 4, un programme d'ordinateur comportant les instructions exécutables par l'unité de traitement 41 de ladite plateforme 4 est chargé dans la mémoire 45, lesdites instructions de programme provoquant la mise en œuvre d'un tel procédé d'intégration et d'exécution 100.

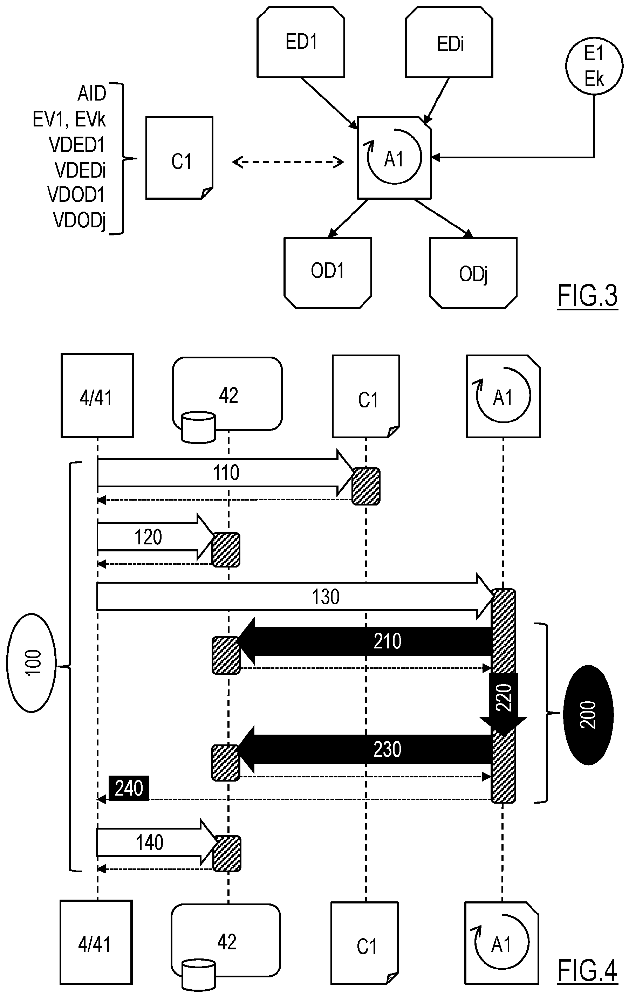

La figure 3 illustre un exemple de description fonctionnelle d'une application logicielle au sens de l'invention. Une telle application logicielle consiste en un traitement numérique portant sur une ou plusieurs données d'entrée pour produire une ou plusieurs données de sortie. A titre d'exemple, une telle première application logicielle peut consister en une application visant à catégoriser un flux d'images DICOM 12 acquises par un appareil d'imagerie à rayons X et produire des données de sortie triées par type, lesdites images étant enrichies par un ou plusieurs labels visant à qualifier lesdites images. De tels label pourraient être choisis parmi un ensemble d'acronymes « CTP », « CTA », « NCCT » d'expressions anglo-saxonnes « *computed tomography perfusion* », « *computed tomography angiography* », « *non-contrast computed tomography* » qualifiant des modalités d'acquisition. En variante, une telle première application logicielle pourrait exploiter des données d'entrée sous la forme d'images DICOM labélisées « CTP » et produire en sortie des données de sortie sous la forme de cartes illustrant un paramètre hémodynamique et un rapport de résultat exprimé sous une forme textuelle et destiné à être communiqué in fine par courriel. Une deuxième application logicielle pourrait être conçue pour exploiter des données d'entrée sous la forme d'un flux 12 d'images DICOM acquises à partir d'un appareil d'imagerie à résonnance magnétique et produire des données de sortie triées par types, lesdites images étant enrichies par un ou plusieurs labels visant à qualifier lesdites images. De tels label pourraient être choisis parmi un ensemble d'acronymes « DWI », « PWI », « FLAIR » d'expressions anglo-saxonnes « *diffusion-weighted imaging* », « *perfusion-weighted imaging* », « *fluid-attenuated inversion recovery* » qualifiant des modalités d'acquisition de l'appareil d'imagerie.

De manière générale et comme l'illustre la figure 3, une application logicielle A1 produit des données de sortie OD1, ..., ODj à partir de données d'entrée ED1, ..., EDi. Lesdites données d'entrée et/ou de sortie peuvent respectivement être de différents formats, par exemple de types textuel, DICOM ou autres. Une application logicielle A1 peut en outre exploiter une ou plusieurs variables d'environnement E1, ..., Ek. De telles variables d'environnement sont transmises à l'application logicielle lors de l'exécution de celle-ci dans le but de paramétrer ladite exécution de l'application logicielle ou les traitements mis en œuvre par celle-ci. Selon un mode de réalisation préférée, l'application A1 peut être définie sous la forme d'un conteneur informatique dont l'image se présente sous la forme d'un fichier statique refermant un code (c'est-à-dire un ensemble d'instructions de programme) exécutable et définissant les paramètres ou dépendances nécessaires à son exécution. Un tel choix technique de « conteneurisation » des applications logicielles permet notamment de simplifier l'étape de test du fonctionnement d'une application logicielle avant son déploiement sans pour autant nécessiter une configuration de test proche d'une plateforme d'imagerie médicale. En effet, le « pattern » de l'application logicielle est clairement défini, l'image du conteneur peut être mise en œuvre et testée telle un module applicatif autonome à l'instar d'une machine virtuelle légère et modulaire. Prenons l'exemple de la technologie « Docker » initialement conçue pour traiter les conteneurs Linux (famille de systèmes d'exploitation « open source » de type Unix (AT&T) créée par Linus Torvalds. Supposons que l'identificateur AID de l'image de l'application logicielle A1 illustrée par la figure 3 désigne la version 1.0 de l'application logicielle « my_application ». Une exécution de ladite application logicielle A1 par un ordinateur pourrait être provoquée en invoquant manuellement la commande :

| | | |
|---|---|---|
| « docker run | | \ |
| | - v /tmp/inputs/ED1:/inputs/ED1 | \ (arg1) |
| | - v /tmp/inputs/ED1:/inputs/EDi | \ (arg2) |
| | - v /tmp/outputs/OD1:/outputs/OD1 | \ (arg3) |
| | - v /tmp/outputs/ODj:/outputs/ODj | \ (arg4) |
| | - e E1=VALUE1 | \ (arg5) |
| | - e Ek=VALUEk | \ (arg6) |
| my_application:1.0 » | | \ (arg7) |

L'argument (arg1) de la commande permet de définir ou résoudre un lien (opération également connue sous le vocable de « mapping » selon une terminologie anglo-saxonne) entre l'emplacement ou répertoire « physique » DED1 de la donnée d'entrée ED1 (en l'espèce selon l'exemple illustré par la figure 2 un répertoire temporaire du système de fichier 45 d'une plateforme 4 d'imagerie médicale ou plus généralement d'un ordinateur de développement ou de test) ledit répertoire DED1 étant accessible par le chemin d'accès DPED1 « /tmp/inputs/ED1 » et le répertoire « virtuel » VDED1 (en l'espèce sur l'exemple illustré par la figure 3, le répertoire virtuel « /inputs/ED1 ») défini par le concepteur de l'application A1 dans le module applicatif. Ainsi, lorsque ladite application logicielle A1 est exécutée, cette dernière lit la valeur de la donnée d'entrée ED1 dans le répertoire virtuel VDED1 « /inputs/ED1 » alors que physiquement, c'est-à-dire dans la mémoire 45 de la machine 4 mettant en œuvre ladite application logicielle A1, ledit répertoire virtuel correspond à un répertoire physique DED1 choisi par ledit utilisateur, en l'espèce le répertoire dont le chemin d'accès DPED1 est « /tmp/inputs/ED1 ».

De la même manière, les arguments (arg2), (arg3) et (arg4) définissent, respectivement pour les données EDi, OD1 et ODj, les liens entre les répertoires virtuels VDEDi « /inputs/EDi », VDOD1 « /outputs/OD1 », VDODj « /outputs/ODj » et les répertoires physiques DEDi « /tmp/inputs/EDi », DOD1 « /outputs/OD1 » et DODj « /outputs/ODj ».

Les arguments (arg5) et (arg6) permettent de transmettre à l'application A1 les valeurs VALUE1 et VALUEk respectivement aux variables d'environnement E1 et Ek.

Enfin l'argument (arg7) de la commande désigne l'identifiant AID de l'image de l'application A1, en l'espèce « my_application » de version 1.0.

L'invention ne saurait être limitée à ce seul choix d'implémentation d'une application logicielle sous la forme d'un conteneur ou de la technologie « Docker ». En variante, elle pourrait s'appuyer sur une solution équivalente telle que la solution « podman » développée par Red Hat. Une application logicielle pourrait également consister en un programme exécutable « classique » en lieu et place d'un conteneur. Dans tous les cas, pour être exploitée par un procédé d'intégration et d'exécution d'applications logicielles conforme à l'invention tel que le procédé 100 dont un exemple de mise en œuvre est illustré par la figure 4, une application logicielle, telle que l'application A1 décrite par la figure 2, est associée à un contenu numérique tiers C1, nommé contrat. Par l'exploitation de ce dernier, par exemple par le module 44 illustré en figure 2, l'exécution d'une application logicielle peut être déclenchée en désignant cette dernière de manière non ambigüe, en résolvant les liens entre les emplacements physiques DED1, DEDi des données d'entrées ED1, EDi, les emplacements physiques DOD1, DODj des données de sortie OD2, ODj sur la plateforme d'imagerie 4 et les emplacements virtuels ou « internes » VDED1, VDEDi, VDOD1, VDODj au module applicatif, voire en définissant des valeurs à des variables d'environnement E1 à Ek. C'est l'exploitation conjointe de ce contrat C1 et du système de fichiers 42 qui permet de faciliter l'intégration et l'exécution d'une application logicielle.

Ainsi, en liaison avec la figure 2 et la figure 3, un contrat C1 comporte un ensemble de champs ou d'éléments, distinguables les uns et d'autres par exemple par des balises ou tout autre moyen technique équivalent, afin que préalablement à l'exécution en tant que telle d'une application logicielle éventuellement paramétrée par des variables d'environnement E1, Ek, les valeurs des données d'entrées ED1, EDi soient inscrites dans des emplacements de la mémoire 45 via le système de fichiers 42 et qu'à l'issue de l'exécution de ladite application logicielle A1, les données de sortie OD1 et ODj produites par celle-ci puissent être exploitées par ladite plateforme 4. Le contrat C1, associé à l'application logicielle A1 peut avantageusement comprendre :
- l'identifiant unique AID de l'application logicielle A1, en l'espèce sur la figure 3, l'image du conteneur ;
- les valeurs EV1 et EVk à assigner respectivement aux variables d'environnement E1 et Ek lors de l'exécution de ladite application logicielle A1;
- les répertoires virtuels d'entrée VDED1, VDEDi destinés à contenir, les valeurs des données d'entrée ED1, EDi de l'application logicielle A1 ;
- les répertoires virtuels de sortie VDOD1, VDODj destinés à contenir les valeurs des données de sortie OD1, ODj de l'application logicielle A1.

Plus généralement, un tel contrat C1 comporte par donnée d'entrée ED1, EDi ou de sortie OD1, ODj, les emplacements virtuels VDED1, VDEDi, VDOD1, VDODj pour écrire/lire une valeur de ladite donnée d'entrée ED1, EDi ou de sortie OD1, ODj. La présence d'une valeur EV1, EVk à assigner à une variable d'environnement est optionnelle, certaines applications logicielles ne nécessitant de tels paramètres de fonctionnement comme, par exemple, pour sélectionner une palette de couleurs parmi une pluralité ou encore un référentiel d'unités. Le format d'un contrat est imposé par la plateforme d'imagerie médicale de sorte que celle-ci puisse avoir la capacité d'exploiter (c'est-à-dire lire et éventuellement mettre à jour) ledit contrat C1. En revanche, le contenu dudit contrat C1 est imposé par l'application logicielle selon les données d'entrée, de sorties/variables d'environnement qu'elle nécessite. Après installation de l'application logicielle et de son contrat en mémoire 45 de la plateforme d'imagerie médicale 4, il revient à l'opérateur de la plateforme ou à la plateforme en tant que telle, de modifier et/ou paramétrer ledit contrat C1 par exemple en inscrivant les chemins d'accès physique DPED1, DPDEi, DPOD1, DPODj aux répertoires DED1, DEDi, DOD1, DODj du système de fichiers 42 régissant la mémoire 45 de ladite plateforme 4 pour stocker les données d'entrée ED1, EDi et de sortie OD1, ODj de ladite application A1 si lesdits répertoires d'entrée DED1, DEDi et de sortie DOD1, DODj sont immuables, ainsi que pour fixer les valeurs de variables d'environnement EV1, EVk si elles existent ou sont requises. De manière préférée, lesdits chemins d'accès DPED1, DPDEi, DPOD1, DPODj ne sont pas inscrits dans le contrat C1 de sorte à laisser la plateforme d'imagerie 4 le soin d'allouer des répertoires temporaires DED1, DEDi, DOD1, DODj préalablement à l'exécution en tant que telle de l'application A1. La résolution des liens entre les répertoires virtuels VDE1, VDEi, VDO1, VDOj et les répertoires physiques DED1, DEDi, DOD1, DODj est réalisée par les arguments de la commande d'exécution comme illustré précédemment en lien avec l'application « my_application ». Nous pouvons parler d'une étape de configuration de l'application logicielle A1 à l'issue de son installation ou chargement en mémoire 45 pour qualifier ladite mise à jour optionnelle du contrat C1.

La figure 4 permet d'illustrer la mise en œuvre et la description fonctionnelle d'un procédé 100 d'intégration et d'exécution d'une application logicielle conforme à l'invention. Un tel procédé 100 est mis en œuvre par une plateforme d'imagerie médicale telle que la plateforme 4 selon la figure 1 et la figure 2. Le fonctionnement de cette dernière est adapté par l'implantation dans la mémoire 45 d'un programme d'ordinateur dont les instructions de programme provoquent, lors de leur exécution par l'unité de traitement 41 sous le contrôle éventuel d'un module décisionnel 44, la mise en œuvre d'un tel procédé 100. Supposons que la mémoire 45 comporte en outre l'application logicielle A1, par exemple sous la forme d'un conteneur associée à un contrat C1, ce dernier se présentant sous la forme d'un fichier texte structuré, c'est-à-dire agencé au moyen de balises idoines, à l'instar des application logicielle A1 et du contrat C1 d'ores et déjà décrits en lien avec la figure 3.

La réception d'une séquence d'images médicales 12 exploitée par l'unité de traitement 41 déclenche la mise en œuvre du procédé 100 pour provoquer l'exécution de l'application logicielle A1 jugée pertinente.

Les principales étapes dudit procédé 100 sont illustrées par des flèches pleines de couleur blanche sur la figure 4. Par ailleurs, les étapes d'un traitement propre à l'application logicielle A1 sont illustrées par des flèches pleines de couleur noire sur ladite figure 4. Cette dernière décrit en outre les interactions entre les « acteurs » principaux concernés par la mise en œuvre dudit procédé 100 (c'est-à-dire l'unité de traitement 41 de la plateforme 4, le système de fichiers 42 opéré par cette dernière, le contrat C1 et l'application logicielle A1).

Ledit procédé 100 comporte une première étape de lecture 110 dans la mémoire 45 du contrat C1 dédié à l'application logicielle A1. Pour rappel cette dernière produit deux données de sortie OD1 et OD2 à partir de données d'entrée ED1 et ED2. Lorsque le contrat C1 comporte les chemins d'accès DPED1, DPEDi à des répertoires DED1, DEDi du système de fichiers 42 qui doivent être exploités pour respectivement initialiser des valeurs des données d'entrées ED1 et EDi, l'unité de traitement 41 connaît en réponse (symbolisée par une flèche en trait fin discontinu) lesdits chemins d'accès DPED1, DPEDi. De la même manière, lorsque le contrat C1 comporte les chemins d'accès DPOD1, DPODj à des répertoires DOD1, DODj du système de fichiers 42 qui doivent être exploités pour respectivement lire des valeurs des données de sortie OD1 et ODj qui seront produites par l'application logicielle A1, l'unité de traitement 41 connaît lesdits chemins d'accès DPOD1, DPODj. Ladite unité de traitement 41 peut également connaître en outre les variables d'environnement E1, Ek éventuellement exploitées par l'application logicielle A1 dont les valeurs EV1, EVk seront transmises lors de l'exécution en tant que telle de ladite application logicielle A1.

En variante, selon un cas plus général, ledit contrat C1 n'a pas fait l'objet de modification ou de configuration par la plateforme d'imagerie médicale 4. L'étape 110 du procédé 100 comporte en outre une sous-étape (non représentée par mesure de simplification sur la figure 4) d'allocation ou de détermination de répertoires temporaires DED1, DEDi du système de fichiers 42 qui doivent être exploités pour respectivement initialiser des valeurs des données d'entrées ED1 et EDi et des répertoires DOD1, DODj du système de fichiers 42 qui doivent être exploités pour respectivement lire des valeurs des données de sortie OD1 et ODj.

Le procédé 100 comporte à présent une étape d'écriture 120 de la valeur de chaque donnée d'entrée ED1, EDi de l'application logicielle A1 dans le répertoire d'entrée DED1, DEDi du système de fichiers 42 tel que spécifié par ledit contrat C1 ou déterminé par la plateforme d'imagerie médicale 4.

Ledit procédé 100 comporte une étape de lancement 130 de l'exécution de l'application logicielle A1 dont l'image est désignée par l'identifiant unique AID tiré de l'étape 110. Une telle étape 130 s'accompagne de la transmission éventuelle de valeurs de variables d'environnement lors que l'application logicielle le requiert, en l'espèce les valeurs EV1 et EVk des variables E1 et Ek. Pour résoudre les liens entre les répertoires d'entrée DED1, DEDi et de sortie DOD1, DODj physiques (c'est-à-dire ceux dont l'accès en lecture/écriture est régi par le système de fichiers) et les répertoires virtuels d'entrée VDED1, VDEDi et de sortie VDOD1, VDODj, l'étape 130 consiste en outre à transmettre les chemins d'accès DPED1, DPEDi, DPOD1, DPODj auxdits répertoires d'entrée DED1, DEDi et de sortie DOD1, DODj pour chaque donnée d'entrée ED1, EDi et de sortie OD1, ODj concernée. Cette étape 130 consiste ainsi à automatiquement constituer un équivalent de la commande manuelle évoquée précédemment en lien avec le test de l'application logicielle « my_application » sous la forme d'un conteneur, les arguments nécessaires à l'exécution de l'application étant transmis à ladite application logicielle conformément aux exigences (éléments, balises et/ou ordre) spécifiées par le contrat C1.

Le procédé 100 demeure en attente du fruit de l'exécution de l'application logicielle A1 (via un compte rendu d'exécution 240 produit par cette dernière, symbolisé par une flèche en trait fin discontinu sur la figure 4). En effet, à l'issue de l'étape 130, l'unité de traitement 41 met en œuvre l'application A1 qui comporte à son tour une première étape 210 visant à lire les valeurs des données d'entrée ED1 et EDi dans les répertoires DED1, DEDi du système de fichiers 42, puis met en œuvre un traitement 220 portant sur lesdites données d'entrée ED1, EDi pour produire des données de sorties OD1, ODj. Les liens entre les répertoires physiques (mémoire 45 / système de fichiers 42) et virtuels ayant été préalablement résolus par les arguments d'exécution transmis l'étape 130 conformément audit contrat C1, les valeurs desdites données de sortie sont inscrites par l'application logicielle A1 en une étape 230 dans les répertoires DOD1, DODj du système de fichiers 42. La mise en œuvre de l'application logicielle A1 se termine par une étape 240 d'élaboration d'un compte rendu d'exécution précisant par exemple si ladite exécution a été réalisée avec ou sans erreur.

Un procédé 100 conforme à l'invention comporte à présent une étape de lecture 140 de la valeur de chaque donnée de sortie OD1, ODj de l'application logicielle A1 dans le répertoire de sortie DOD1, DODj du système de fichiers 42 tel que défini par le contrat C1 ou par la plateforme 4.

A l'issue de la mise en œuvre du procédé d'intégration et d'exécution de l'application logicielle A1, la plateforme d'imagerie médicale 4 peut déclencher la mise en œuvre d'un traitement subséquent visant à exploiter le résultat produit par ladite application A1. Un tel traitement subséquent peut consister en une nouvelle occurrence dudit procédé 100 pour provoquer l'exécution d'un deuxième application logicielle A2, An.

A titre avantageux, l'invention prévoit qu'une application logicielle A1, A2, An puisse gérer une donnée d'entrée ou une donnée de sortie présentant un caractère optionnel. Pour cela, le caractère optionnel peut être spécifié dans le contrat C1, C2, Cn qui est associé à ladite application A1, A2, An. Selon un mode de réalisation préféré, en liaison avec l'application A1 illustrée par la figure 3, le champ ou élément dudit contrat C1 déterminant le répertoire virtuel VDED1, VDEDi destiné à contenir une donnée d'entrée ED1, EDi peut être associé à un attribut précisant un caractère optionnel ou obligatoire de ladite donnée d'entrée ED1, EDi. Selon ce mode de réalisation, un procédé 100 selon l'invention est adapté de sorte que, lorsqu'un tel attribut atteste un caractère optionnel d'une donnée d'entrée ED1, EDi, l'étape d'écriture 120 de la valeur de cette dernière n'est mise en œuvre que si une telle donnée d'entrée ED1, EDi est disponible pour la plateforme d'imagerie médicale 4. Le lancement de l'exécution de l'application logicielle A1 ne provoquera pas d'erreur en l'absence d'argument dédié à une telle donnée optionnelle dans la commande 130. Un tel attribut peut ainsi consister en une valeur booléenne pour caractériser un tel caractère optionnel ou obligatoire d'une donnée d'entrée. En variante, l'absence d'un tel attribut peut signifier que ladite donnée d'entrée revêt un caractère obligatoire.

Il en est de même pour une donnée de sortie OD1, ODj qui peut être optionnelle selon le résultat produit par une application logicielle telle que l'application A1 selon la figure 3. Selon ce mode de réalisation, le champ ou élément dudit contrat C1 déterminant le répertoire virtuel VDOD1, VDODj destiné à contenir une donnée de sortie OD1, ODi peut être associé à un attribut précisant le caractère optionnel ou obligatoire de ladite donnée de sortie OD1, ODj. Lorsqu'un tel attribut atteste un caractère optionnel de ladite donnée de sortie OD1, ODj, ce dernier peut comporter une valeur déterminée spécifiant une valeur de non-intérêt. De cette manière, l'unité de traitement 41 sait qu'il ne faut pas tenir compte d'une telle donnée de sortie si ladite donnée décrit une valeur égale à ladite valeur déterminée.

Par ailleurs, l'invention prévoit d'ajouter une ou des méta-informations, telles qu'un label ou une étiquette ou, plus généralement un attribut de typage pour qualifier une donnée d'entrée ED1, EDi et/ou de sortie OD1, ODj d'une application logicielle telle que l'application A1 selon la figure 3. Lorsqu'une telle donnée de sortie OD1, ODj peut être enrichie ou qualifiée par la présence d'un label, le contrat C1 associé à ladite application logicielle A1 peut être agencé de sorte que le répertoire virtuel de sortie VDOD1, VDODj destiné à contenir la valeur de la donnée de sortie, puisse être associé à un tel attribut de typage de ladite donnée de sortie OD1, ODj. Dans ce cas, l'étape de lecture 140 (du procédé 100 selon la figure 4) de la valeur de la donnée de sortie OD1, ODj concernée peut consister à associer ladite valeur dudit attribut de typage à la valeur de ladite donnée de sortie. Lorsque l'application logicielle A1 ne prévoit pas un tel typage, le contrat C1 qui lui est associé n'associe pas un tel attribut de typage. En revanche, la plateforme d'imagerie 4 peut mettre à jour ledit contrat C1 pour adjoindre ledit attribut de typage au répertoire virtuel de sortie de la donnée de sortie concernée. Un tel enrichissement d'une donnée de sortie produite par une application logicielle peut ainsi être mis à profit par la plateforme d'imagerie médicale 4 pour choisir des données d'entrée ED1, EDi de l'application logicielle A1 à inscrire dans le répertoire d'entrée DED1, DEDi du système de fichiers 42 ou pour choisir des données d'entrée de toute autre application.

L'invention prévoit en outre un mode de réalisation avantageux selon lequel un contrat C1, C2, Cn associé à une application logicielle A1, A2, An, peut être mis à jour ou initialisé de manière sécurisée. A ce titre, un tel contrat peut être chiffré après toute modification et déchiffré durant l'étape 110 d'un procédé 100 selon l'invention et tel qu'illustré par la figure 4. L'identifiant AID de l'application logicielle A1 peut en outre comporter un code de redondance (par exemple, un haché de ladite application logicielle associée) de sorte que l'étape 130 de lancement de l'exécution de l'application comporte un traitement pour vérifier ce code de redondance préalablement au déclenchement de l'exécution en tant que telle de l'application logicielle. Si le code de redondance calculé correspond à la valeur inscrite dans le contrat, l'exécution de l'application logicielle est confirmée et provoquée. Dans le cas contraire, ladite exécution est avortée. De cette manière, la plateforme d'imagerie médicale 4 peut s'assurer que le contrat est bien associé avec la version idoine de ladite application logicielle.

## Revendications

1. Procédé (100) d'intégration et d'exécution d'une application logicielle (A1, A2, An), ledit procédé étant mis en œuvre par une unité de traitement (41) d'une plateforme d'imagerie médicale (4) opérant un système de fichiers (42), ledit procédé (100) comportant :
- une étape de chargement d'une application logicielle (A1, A2, An) dans une mémoire (45) de la plateforme d'imagerie médicale (4) ;
- une étape d'écriture (120) de la valeur de la donnée d'entrée (ED1, EDi) de l'application logicielle (A1, A2, An) dans un répertoire d'entrée (DED1, DEDi) du système de fichiers (42) ;
- une étape de lancement (130) de l'exécution de l'application logicielle (A1, A2, An) ;
- une étape de lecture (140) de la valeur de la donnée de sortie (OD1, ODj) de l'application logicielle (A1, A2, An) dans un répertoire de sortie (DOD1, DODj) du système de fichiers (42) ;
ledit procédé étant **caractérisé en ce que** :
- ledit procédé comporte en outre :
a. une étape de chargement d'un contrat (C1, C2, Cn) dédié à ladite application logicielle (A1, A2, An) dans ladite mémoire (45) de la plateforme d'imagerie médicale (4), ledit contrat (C1, C2, Cn) consistant en un contenu numérique non modifié ou configuré par ladite plateforme d'imagerie médicale (4), qui comporte des informations exploitables par cette dernière (4) et imposées par l'application logicielle (A1, A2, An), lesdites informations décrivant :
i. un identifiant unique (AID) désignant ladite application logicielle (A1, A2, A3) qui est ainsi associée au contrat (C1, C2, Cn) qui lui est dédié,
ii. un répertoire virtuel d'entrée (VDED1, VDEDi) destiné à contenir une valeur d'une donnée d'entrée (ED1, EDi) nécessaire à l'exécution de l'application logicielle (A1, A2, An) ;
iii. un répertoire virtuel de sortie (VDOD1, VDODj) destiné à contenir la valeur de la donnée de sortie (OD1, ODj) produite par cette dernière,
ladite étape de chargement dudit contrat (C1, C2, Cn) étant réalisée conjointement avec le chargement de l'application logicielle (A1, A2, An) qui lui est associée dans la mémoire (45) de la plateforme d'imagerie médicale (4) ;
b. une étape de lecture (110) du contrat (C1, C2, Cn) dédié à l'application logicielle (A1, A2, An) qui lui est associée par la présence de l'identifiant unique (AID) de cette dernière inscrit dans ledit contrat (C1, C2, Cn), pour connaître les exigences de ladite application logicielle (A1, A2, An) pour être exécutée, ladite étape de lecture (110) du contrat (C1, C2, Cn) précédant l'étape de lancement (130) de l'exécution de ladite application logicielle (A1, A2, An) ;
- l'application logicielle (A1, A2, An) est un code exécutable conçu et produit indépendamment de la mise en œuvre du présent procédé (100) d'intégration et d'exécution ou de tout autre procédé mis en œuvre par l'unité de traitement (41) de ladite plateforme d'imagerie médicale (4) ;
- l'étape de lancement (130) de l'exécution de l'application logicielle (A1, A2, An) désignée par ledit identifiant unique (AID) tiré du contrat (C1, C2, Cn) lu préalablement (110), comprend une transmission d'arguments d'exécution de l'application logicielle (A1, A2, An) consistant en des chemins d'accès (DPED1, DPEDi, DPOD1, DPODj) au répertoire d'entrée (DED1, DEDi) du système de fichiers (42) et au répertoire de sortie (DOD1, DODj) dudit système de fichiers (42), lesdits chemins d'accès (DPED1, DPEDi, DPOD1, DPODj) étant ainsi associés respectivement aux répertoires virtuels d'entrée (VDED1, VDEDi) et de sortie (VDOD1, VDODj) de l'application logicielle (A1, A2, An) grâce auxdits arguments d'exécution transmis à l'application logicielle (A1, A2, An) à l'étape de lancement (130) de l'exécution de cette dernière (A1, A2, An) conformément au contenu du contrat (C1, C2, Cn) préalablement lu à l'étape de lecture (110) dudit contrat (C1, C2, Cn) dédié à ladite application logicielle (A1, A2, An).

2. Procédé (100) selon la revendication précédente, pour lequel le contrat (C1, C2, Cn) comporte une valeur (EV1, EVk) d'un paramètre de fonctionnement (E1, Ek) de l'application logicielle (A1, A2, An) qui lui est associée et pour lequel l'étape de lancement (130) de l'exécution de ladite application logicielle (A1, A2, An) est adaptée pour comporter la transmission de la valeur dudit paramètre de fonctionnement en argument d'exécution de l'application logicielle (A1, A2, An) conformément au contenu du contrat (C1, C2, Cn) lu préalablement à l'étape de lecture (110) dudit contrat (C1, C2, Cn) dédié à l'application logicielle (A1, A2, An).

3. Procédé selon l'une quelconque des revendications précédentes, pour lequel l'application logicielle (A1, A2, An) se présente sous la forme d'un conteneur informatique, l'identifiant unique (AID) désignant ladite application logicielle (A1, A2, An) compris dans ledit contrat (C1, C2, Cn) qui lui est associé, exprimant l'image dudit conteneur informatique.

4. Procédé (100) selon l'une quelconque des revendications précédentes pour lequel :
- le répertoire virtuel d'entrée (VDED1, VDEDi) qui est destiné à contenir la donnée d'entrée (ED1, EDi) de l'application logicielle (A1, A2, An) est associé à un attribut précisant le caractère optionnel ou obligatoire de ladite donnée d'entrée (ED1, EDi) dans le contrat (C1, C2, Cn) qui est dédié à ladite application logicielle (A1, A2, An) et
- l'étape d'écriture (120) de cette dernière n'est mise en œuvre que si une telle donnée d'entrée (ED1, EDi) est disponible pour la plateforme d'imagerie médicale (4) lorsqu'un tel attribut atteste un caractère optionnel de ladite donnée d'entrée (ED1, EDi).

5. Procédé (100) selon l'une quelconque des revendications précédentes pour lequel :
- le répertoire virtuel de sortie (DOD1, DODj) destiné à contenir la valeur de la donnée de sortie (OD1, ODj) de l'application logicielle (A1, A2, An), est associé à un attribut de typage de ladite sortie (OD1, ODj) dans le contrat (C1, C2, Cn) qui est dédié à ladite application logicielle (A1, A2, An) et
- l'étape de lecture (140) de la valeur de la donnée de sortie (OD1, ODj) de l'application logicielle (A1, A2, An) consiste à associer ladite valeur dudit attribut de typage à la valeur de ladite donnée de sortie (OD1, ODj).

6. Procédé (100) selon l'une quelconque des revendications précédentes pour lequel :
- le contrat (C1, C2, Cn) qui est dédié à une application logicielle (A1, A2, An) est encodé préalablement à l'intégration de cette dernière dans la mémoire (45) de la plateforme d'imagerie médicale (4) et pour lequel l'étape de lecture (110) d'un tel contrat (C1, C2, Cn) comporte une sous-étape préalable de décodage dudit contrat (C1, C2, Cn).

7. Produit programme d'ordinateur comportant une ou plusieurs instructions de programme exécutables par l'unité de traitement (41) d'un ordinateur, lesdites instructions de programme étant chargeables dans une mémoire non volatile (45) dudit ordinateur et dont l'exécution par ladite unité de traitement (41) provoque la mise en œuvre d'un procédé (100) selon l'une quelconque des revendications précédentes.

8. Support de mémorisation lisible par un ordinateur comportant les instructions d'un produit programme d'ordinateur selon la revendication précédente.

9. Plateforme d'imagerie médicale (4) comprenant une unité de traitement (41), une mémoire (45) exploitée par un système de fichiers (42), ladite mémoire (45) comprenant les instructions de programme d'un produit programme d'ordinateur selon la revendication 7.

## Patentansprüche

1. Verfahren (100) zum Integrieren und Ausführen einer Softwareanwendung (A1, A2, An), wobei dieses Verfahren durch eine Verarbeitungseinheit (41) einer ein Dateisystem (42) betreibenden medizinischen Bildgebungsplattform (4) implementiert wird, wobei dieses Verfahren (100) umfasst:
- einen Schritt des Ladens einer Softwareanwendung (A1, A2, An) in einen Speicher (45) der medizinischen Bildgebungsplattform (4);
- einen Schritt des Schreibens (120) des Wertes des Eingabedatenelements (ED1, EDi) der Softwareanwendung (A1, A2, An) in ein Eingabeverzeichnis (DED1, DEDi) des Dateisystems (42);
- einen Schritt des Startens (130) der Ausführung der Softwareanwendung (A1, A2, An);
- einen Schritt des Lesens (140) des Wertes des Ausgabedatenelements (OD1, ODj) der Softwareanwendung (A1, A2, An) in einem Ausgabeverzeichnis (DOD1, DODj) des Dateisystems (42);
wobei das Verfahren **dadurch gekennzeichnet ist, dass:**
- dieses Verfahren ferner umfasst:
a. einen Schritt des Ladens eines der Softwareanwendung (A1, A2, An) zugeordneten Vertrags (C1, C2, Cn) in diesen Speicher (45) der medizinischen Bildgebungsplattform (4), wobei der Vertrag (C1, C2, Cn) aus einem digitalen Inhalt besteht, der von dieser medizinischen Bildgebungsplattform (4) weder verändert noch konfiguriert wird, der Informationen enthält, die durch diese (4) verwendbar sind und durch die Softwareanwendung (A1, A2, An) vorgegeben werden, wobei diese Informationen Folgendes beschreiben:
i. eine eindeutige Kennung (AID) zur Bezeichnung dieser Softwareanwendung (A1, A2, A3), die somit mit dem ihr zugeordneten Vertrag (C1, C2, Cn) verknüpft ist,
ii. ein virtuelles Eingabeverzeichnis (VDED1, VDEDi), das einen Wert eines Eingabedatenelements (ED1, EDi) enthalten soll, das für die Ausführung der Softwareanwendung (A1, A2, An) erforderlich ist;
iii. ein virtuelles Ausgabeverzeichnis (VDOD1, VDODj), das den Wert des von diesem erzeugten Ausgabedatenelements (OD1, ODj) enthalten soll,
wobei dieser Schritt des Ladens dieses Vertrags (C1, C2, Cn) zusammen mit dem Laden der mit ihm verknüpften Softwareanwendung (A1, A2, An) in den Speicher (45) der Bildgebungsplattform (4) erfolgt;
b. einen Schritt des Lesens (110) des Vertrags (C1, C2, Cn), der der Softwareanwendung (A1, A2, An) zugeordnet ist, die mit ihm durch das Vorhandensein der in diesen Vertrag (C1, C2, Cn) eingetragenen eindeutigen Kennung (AID) davon verknüpft ist, um die Anforderungen dieser Softwareanwendung (A1, A2, An) zu deren Ausführung zu erkennen, wobei dieser Schritt des Lesens (110) des Vertrags (C1, C2, Cn) dem Schritt des Startens (130) der Ausführung der Softwareanwendung (A1, A2, An) vorausgeht;
- die Softwareanwendung (A1, A2, An) ein ausführbarer Code ist, der unabhängig von der Implementierung dieses Integrations- und Ausführungsverfahrens (100) oder eines anderen Verfahrens, das von der Verarbeitungseinheit (41) dieser medizinischen Bildgebungsplattform (4) implementiert wird, konzipiert und erzeugt wurde;
- der Schritt des Startens (130) der Ausführung der Softwareanwendung (A1, A2, An), die durch diese aus dem zuvor gelesenen Vertrag (C1, C2, Cn) entnommene eindeutige Kennung (AID) (110) gekennzeichnet ist, eine Übermittlung von Ausführungsargumenten der Softwareanwendung (A1, A2, An) umfasst, die aus Zugriffspfaden (DPED1, DPEDi, DPOD1, DPODj) zum Eingabeverzeichnis (DED1, DEDi) des Dateisystems (42) und zum Ausgabeverzeichnis (DOD1, DODj) dieses Dateisystems (42) bestehen, wobei diese Zugriffspfade (DPED1, DPEDi, DPOD1, DPODj) somit mit den virtuellen Eingabe- (VDED1, VDEDi) bzw. Ausgabeverzeichnissen (VDOD1, VDODj) der Softwareanwendung (A1, A2, An) verknüpft sind, und zwar mittels der Ausführungsargumente, die der Softwareanwendung (A1, A2, An) im Schritt des Startens (130) von deren Ausführung (A1, A2, An) entsprechend dem Inhalt des Vertrags (C1, C2, Cn), der zuvor im Schritt des Lesens (110) des der Softwareanwendung (A1, A2, An) zugeordneten Vertrags (C1, C2, Cn) gelesen wurde, übermittelt werden.

2. Verfahren (100) nach dem vorstehenden Anspruch, wobei der Vertrag (C1, C2, Cn) einen Wert (EV1, EVk) eines Betriebsparameters (E1, Ek) der damit verknüpften Softwareanwendung (A1, A2, An) enthält und wobei der Schritt des Startens (130) der Ausführung dieser Softwareanwendung (A1, A2, An) so ausgelegt ist, dass die Übermittlung des Wertes des Betriebsparameters als Ausführungsargument der Softwareanwendung (A1, A2, An) gemäß dem Inhalt des Vertrags (C1, C2, Cn) erfolgt, der vor dem Schritt des Lesens (110) des der Softwareanwendung (A1, A2, An) zugeordneten Vertrags (C1, C2, Cn) gelesen wird.

3. Verfahren nach einem der vorstehenden Ansprüche, wobei die Softwareanwendung (A1, A2, An) in Form eines Computercontainers vorliegt, wobei die eindeutige Kennung (AID), die diese Softwareanwendung (A1, A2, An) bezeichnet, die in dem mit dieser verknüpften Vertrag (C1, C2, Cn) enthalten ist, das Bild des Computercontainers ausdrückt.

4. Verfahren (100) nach einem der vorstehenden Ansprüche, wobei:
- das virtuelle Eingabeverzeichnis (VDED1, VDEDi), das das Eingabedatenelement (ED1, EDi) der Softwareanwendung (A1, A2, An) enthalten soll, mit einem Attribut verknüpft ist, das angibt, ob dieses Eingabedatenelement (ED1, EDi) im Vertrag (C1, C2, Cn), der dieser Softwareanwendung (A1, A2, An) zugeordnet ist, einen optionalen oder obligatorischen Charakter aufweist, und
- der Schritt des Schreibens (120) von diesem nur dann implementiert wird, wenn ein solches Eingabedatenelement (ED1, EDi) für die medizinische Bildgebungsplattform (4) verfügbar ist, wenn ein solches Attribut einen optionalen Charakter des genannten Eingabedatenelements (ED1, EDi) bestätigt.

5. Verfahren (100) nach einem der vorstehenden Ansprüche, wobei:
- das virtuelle Ausgabeverzeichnis (DOD1, DODj), das den Wert des Ausgabedatenelements (OD1, ODj) der Softwareanwendung (A1, A2, An) enthalten soll, mit einem Typisierungsattribut dieser Ausgabe (OD1, ODj) im Vertrag (C1, C2, Cn) verknüpft ist, der dieser Softwareanwendung (A1, A2, An) zugeordnet ist, und
- der Schritt des Lesens (140) des Wertes des Ausgabedatenelements (OD1, ODj) der Softwareanwendung (A1, A2, An) darin besteht, den Wert dieses Typisierungsattributs mit dem Wert des Ausgabedatenelements (OD1, ODj) zu verknüpfen.

6. Verfahren (100) nach einem der vorstehenden Ansprüche, wobei:
- der Vertrag (C1, C2, Cn), der einer Softwareanwendung (A1, A2, An) zugeordnet ist, vor deren Integration in den Speicher (45) der medizinischen Bildgebungsplattform (4) codiert wird, und wobei der Schritt des Lesens (110) eines solchen Vertrags (C1, C2, Cn) einen vorhergehenden Teilschritt der Decodierung dieses Vertrags (C1, C2, Cn) umfasst.

7. Computerprogrammprodukt, umfassend eine oder mehrere Programmanweisungen, die durch die Verarbeitungseinheit (41) eines Computers ausführbar sind, wobei diese Programmanweisungen in einen nichtflüchtigen Speicher (45) des Computers ladbar sind und deren Ausführung durch die Verarbeitungseinheit (41) die Implementierung eines Verfahrens (100) nach einem der vorstehenden Ansprüche bewirkt.

8. Computerlesbares Speichermedium, umfassend die Anweisungen eines Computerprogrammprodukts nach dem vorstehenden Anspruch.

9. Medizinische Bildgebungsplattform (4), umfassend eine Verarbeitungseinheit (41), einen Speicher (45), der von einem Dateisystem (42) verwendet wird, wobei der Speicher (45) die Programmanweisungen eines Computerprogrammprodukts nach Anspruch 7 umfasst.

## Claims

1. Method (100) for integrating and executing a software application (A1, A2, An), said method being implemented by a processing unit (41) of a medical imaging platform (4) operating a file system (42), said method (100) comprising:
- a step of loading a software application (A1, A2, An) in a memory (45) of the medical imaging platform (4);
- a step (120) of writing the value of the item of input data (ED1, EDi) of the software application (A1, A2, An) to an input directory (DED1, DEDi) of the file system (42);
- a step (130) of initializing the execution of the software application (A1, A2, An);
- a step (140) of reading the value of the item of output data (OD1, ODj) of the software application (A1, A2, An) from an output directory (DOD1, DODj) of the file system (42);
said method being **characterized in that:**
- said method further comprises:
a. a step of loading a contract (C1, C2, Cn) dedicated to said software application (A1, A2, An) in said memory (45) of the medical imaging platform (4), said contract (C1, C2, Cn) consisting of digital content not modified or configured by said medical imaging platform (4), which includes information usable by the medical imaging platform (4) and imposed by the software application (A1, A2, An), said information describing:
i. a unique identifier (AID) denoting said software application (A1, A2, A3) which is thus associated with the contract (C1, C2, Cn) which is dedicated to said application,
ii. an input virtual directory (VDED1, VDEDi) intended to contain a value of an item of input data (ED1, EDi) required to execute the software application (A1, A2, An);
iii. an output virtual directory (VDOD1, VDODj) intended to contain the value of the item of output data (OD1, ODj) produced by said application,
said step of loading said contract (C1, C2, Cn) being carried out together with the loading of the software application (A1, A2, An) which is associated therewith in the memory (45) of the medical imaging platform (4);
b. a step (110) of reading the contract (C1, C2, Cn) dedicated to the software application (A1, A2, An) which is associated therewith by the presence of the unique identifier (AID) of the software application written in said contract (C1, C2, Cn), in order to know the requirements of said software application (A1, A2, An) to be executed, said step (110) of reading the contract (C1, C2, Cn) preceding the step (130) of initializing the execution of said software application (A1, A2, An);
- the software application (A1, A2, An) is an executable code designed and produced independently of the implementation of the present integration and execution method (100) or of any other method implemented by the processing unit (41) of said medical imaging platform (4);
- the step (130) of initializing the execution of the software application (A1, A2, An) denoted by said unique identifier (AID) taken from the contract (C1, C2, Cn) previously read (110), comprises transmitting arguments for executing the software application (A1, A2, An) consisting of access paths (DPED1, DPEDi, DPOD1, DPODj) to the input directory (DED1, DEDi) of the file system (42) and to the output directory (DOD1, DODj) of said file system (42), said access paths (DPED1, DPEDi, DPOD1, DPODj) are thus associated with the input virtual directory (VDED1, VDEDi) and output virtual directory (VDOD1, VDODj), respectively, of the software application (A1, A2, An) by virtue of said execution arguments transmitted to the software application (A1, A2, An) in the step (130) of initializing the execution of the software application (A1, A2, An) in accordance with the content of the contract (C1, C2, Cn) previously read in the step of reading (110) said contract (C1, C2, Cn) dedicated to said software application (A1, A2, An).

2. Method (100) according to the preceding claim, wherein the contract (C1, C2, Cn) comprises a value (EV1, EVk) of an operating parameter (E1, Ek) of the software application (A1, A2, An) associated therewith and wherein the step (130) of initializing the execution of said software application (A1, A2, An) is suitable for including transmission of the value of said operating parameter as an argument for executing the software application (A1, A2, An) in accordance with the content of the contract (C1, C2, Cn) read prior to the step (110) of reading said contract (C1, C2, Cn) dedicated to the software application (A1, A2, An).

3. Method according to any one of the preceding claims, wherein the software application (A1, A2, An) is in the form of a computer container, the unique identifier (AID) denoting said software application (A1, A2, An) included in said contract (C1, C2, Cn) which is associated therewith, expressing the image of said computer container.

4. Method (100) according to any one of the preceding claims, wherein:
- the input virtual directory (VDED1, VDEDi) which is intended to contain the item of input data (ED1, EDi) of the software application (A1, A2, An) is associated with an attribute specifying the optional or mandatory nature of said item of input data (ED1, EDi) in the contract (C1, C2, Cn) which is dedicated to said software application (A1, A2, An), and
- the writing step (120) of the software application is implemented only if such an item of input data (ED1, EDi) is available for the medical imaging platform (4) when such an attribute attests to an optional nature of said item of input data (ED1, EDi).

5. Method (100) according to any one of the preceding claims, wherein:
- the output virtual directory (DOD1, DODj) intended to contain the value of the item of output data (OD1, ODj) of the software application (A1, A2, An) is associated with a typing attribute of said output (OD1, ODj) in the contract (C1, C2, Cn) which is dedicated to said software application (A1, A2, An), and
- the step (140) of reading the value of the item of output data (OD1, ODj) of the software application (A1, A2, An) consists in associating said value of said typing attribute with the value of said item of output data (OD1, ODj).

6. Method (100) according to any one of the preceding claims, wherein:
- the contract (C1, C2, Cn) which is dedicated to a software application (A1, A2, An) is encoded prior to integrating the software application in the memory (45) of the medical imaging platform (4) and wherein the step (110) of reading such a contract (C1, C2, Cn) comprises a prior sub-step of decoding said contract (C1, C2, Cn).

7. Computer program product comprising one or more program instructions which can be executed by the processing unit (41) of a computer, said program instructions being loadable into a non-volatile memory (45) of said computer and the execution of said instructions by said processing unit (41) causes the implementation of a method (100) according to any one of the preceding claims.

8. Computer-readable storage medium comprising the instructions of a computer program product according to the preceding claim.

9. Medical imaging platform (4) comprising a processing unit (41), a memory (45) operated by a file system (42), said memory (45) comprising the program instructions of a computer program product according to claim 7.
